## Europäisches Patentamt

## European Patent Office

## Office européen des brevets

(11) Numéro de publication: **0 208 154**
**B1**

(12) **FASCICULE DE BREVET EUROPÉEN**

(45) Date de publication du fascicule du brevet:
**13.09.89**

(21) Numéro de dépôt: **86107827.7**

(22) Date de dépôt: **09.06.86**

(51) Int. Cl.⁴: **A 61 N 1/05**

(54) **Electrode implantable.**

(30) Priorité: **11.06.85 FR 8508932**

(43) Date de publication de la demande:
**14.01.87 Bulletin 87/3**

(45) Mention de la délivrance du brevet:
**13.09.89 Bulletin 89/37**

(84) Etats contractants désignés:
**CH DE GB LI NL SE**

(56) Documents cité:
**EP-A-0 042 551**
**EP-A-0 126 981**
**DE-A-2 949 782**
**FR-A-2 091 400**
**FR-A-2 565 111**
**US-A-4 236 529**
**US-A-4 280 514**

(73) Titulaire: **Nivarox- FAR S.A., Avenue du Collège 10, CH- 2400 Le Locle (CH)**

(72) Inventeur: **Maillard, Germain, Crêtets 10, CH- 2300 La Chaux- de- Fonds (CH)**
Inventeur: **Meyrat, Pierre- André, Grand- Cernil 3, CH- 2416 Les Brenets (CH)**

(74) Mandataire: **Caron, Gérard, ICB Ingénieurs Conseils en Brevets SA Passage Max. Meuron 6, CH- 2001 Neuchâtel (CH)**

## Description

La présente invention est relative aux électrodes implantables destinées à transmettre de l'énergie électrique à un organe du corps. Plus particulièrement, l'invention concerne les électrodes endocardiaques qui assurent la connexion électrique entre un stimulateur et le muscle cardiaque.

Dans le brevet CH-570 804, on décrit une telle électrode dont la tête peut présenter une section transversale en forme d'étoile afin de créer sur cette tête des cavités dans lesquelles le tissu cardiaque peut croître afin d'assurer après l'implantation de l'électrode un bon accrochage de celle-ci sur le muscle cardiaque.

Par ailleurs, dans ce brevet, la tête de l'électrode est conformée de telle manière que la densité de courant aux parties conductrices en contact avec le tissu cardiaque soit aussi élevée que possible.

C'est pourquoi les parois des cavités formées sur la tête sont revêtues d'un isolant sur la majeure partie de leur surface pour réduire la surface de contact et ainsi augmenter la densité de courant, l'énergie étant transmise par un grand nombre de petits éléments conducteurs.

Dans le brevet FR-2 345 167, on décrit également une électrode de stimulation cardiaque qui comporte une tête formée, d'une part, d'une extrémité en carbone destinée à abaisser le seuil d'excitation d'un battement cardiaque et, d'autre part, une pièce cylindrique séparée de l'extrémité en carbone par un matériau isolant et réalisée en un matériau auquel adhère facilement le tissu cardiaque. La pièce cylindrique peut être en un matériau poreux ou en plusieurs éléments annulaires séparés par des entretoises de moindre diamètre afin de créer des cavités annulaires dans lesquelles peut croître le tissu pour améliorer l'accrochage.

Toutefois, aucune des électrodes proposées par les deux brevets précités n'apporte une solution véritablement satisfaisante. En effet, en ce qui concerne le brevet suisse, on a observé que l'efficacité de la transmission de l'énergie au muscle cardiaque pouvait être grandement améliorée non pas en augmentant la densité de courant, mais au contraire en augmentant la surface de contact électrique, c'est-à-dire en réduisant la résistance de contact au niveau de l'interface électrode - tissu. Le fait, par ailleurs, que dans ce brevet, l'électrode comporte des rainures axiales ne favorise pas beaucoup l'accrochage étant donné que les forces de décrochage du tissu agissent principalement axialement sur le conducteur et la tête de l'électrode.

On peut remarquer, par ailleurs, en ce qui concerne le brevet français précité que le fait de prévoir dans la tête de l'électrode plusieurs éléments distincts tels que l'extrémité en carbone et les éléments annulaires successifs ne facilite pas la fabrication et une telle électrode implique donc un prix de revient élevé.

L'invention a pour but de fournir une électrode implantable qui allie une bonne adhérence au tissu de l'organe à desservir à une très faible résistance électrique, tout en présentant par ailleurs un prix de revient faible grâce à la facilité avec laquelle elle peut être fabriquée.

Le brevet EP-A-0 126 981 décrit une électrode à faible seuil et à faible polarisation dont l'extrémité distale comporte deux rainures circulaires à section en forme de V agencées coaxialement sur la tête de l'électrode. Les rainures sont prévues pour contribuer à assurer la fonction de l'électrode à l'endocarde par la formation d'invaginations dans lesquelles le tissu peut croître, ce qui évite le déplacement de l'électrode par rapport à l'endocarde et maintient le seuil de déclenchement à une valeur acceptable.

L'invention a donc pour objet une électrode implantable, notamment endocardiaque, destinée à établir une connexion avec un organe du corps, qui comporte un conducteur isolé, à l'extrémité distale duquel est prévue une tête conductrice destinée à s'accrocher au tissu à desservir pour y assurer ladite connexion, la surface extérieure de la tête étant de révolution autour de l'axe longitudinal du conducteur isolé, caractérisée en ce que ladite surface extérieure présente un réseau de rainures croisées venues de formage dans le corps de la tête.

Grâce à cette caractéristique, l'électrode présente comparativement une surface de contact considérable, c'est-à-dire une faible impédance au niveau de l'interface avec le tissu, tandis que par ailleurs, les rainures croisées constituent autant de cavités dans lesquelles le tissu peut adhérer en formant une sorte d'ensemble gaufré, qui retient l'électrode tant axialement que suivant la circonférence de la tête.

Suivant une autre caractéristique de l'invention, il peut être avantageux pour augmenter encore davantage le pouvoir adhérant, de conférer aux rainures croisées une section en forme de trapèze dont les branches latérales s'évasent vers l'extérieur.

Plusieurs formes de la tête d'électrode peuvent être envisagées telles que la forme sphérique, cylindrique à section circulaire, conique, biconique ou analogue.

De préférence, le croisement des rainures est prévu à angle droit, mais il est également possible de prévoir une rainure hélicoïdale dont l'hélice a un axe qui coïncide avec l'axe de la tête et qui se croise avec des rainures longitudinales réparties autour de cette tête.

L'invention sera mieux comprise à la lecture de la description qui va suivre, faite en référence aux dessins annexés, donnés uniquement à titre d'exemple et sur lesquels :

- la figure 1 est une vue partielle en coupe axiale d'une électrode implantable suivant l'invention;
- la figure 2 est une vue en coupe radiale, à une échelle agrandie, suivant la ligne II - II de la figure 1;
- la figure 3 montre une vue en coupe axiale d'une variante de l'électrode;

- les figures 4 à 7 illustrent plusieurs formes possibles de la tête de l'électrode et de son réseau de rainures croisées.

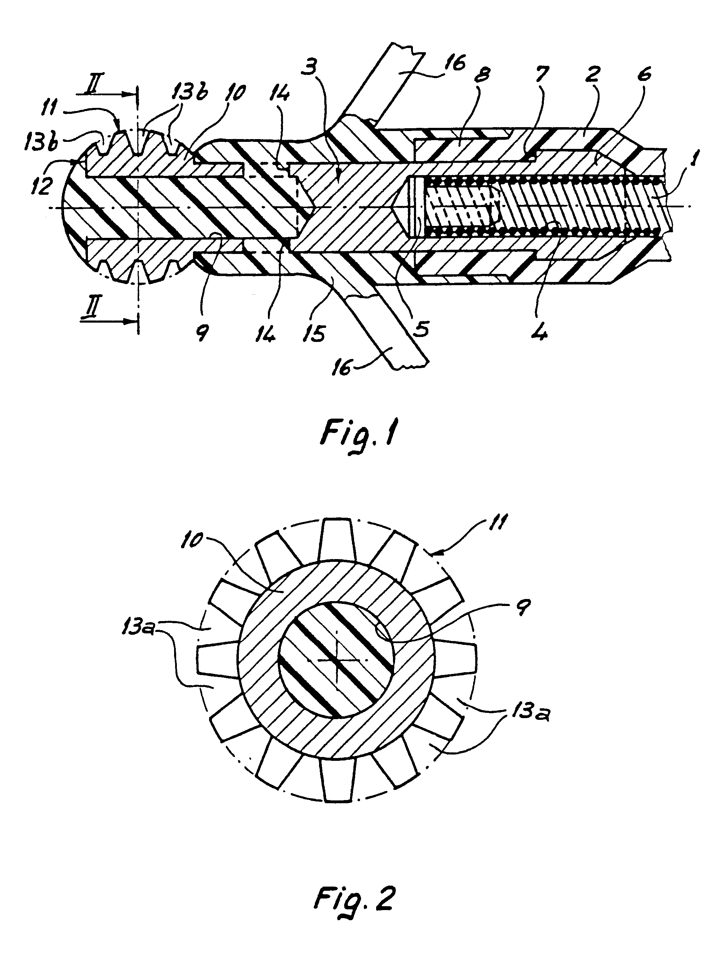

Les figures 1 et 2 montrent que l'électrode implantable comporte un conducteur 1 qui, de façon connue en soi, est formée d'un enroulement hélicoïdal de plusieurs torons de fil mince eux-mêmes enroulés en hélice. Ce conducteur est destiné à être relié à un appareil d'où provient l'énergie à transmettre à l'organe du corps. Par exemple, s'il s'agit d'une électrode endocardiaque, celle-ci peut être reliée à un stimulateur cardiaque implanté ou autonome (en milieu hospitalier, par exemple). La connexion avec l'appareil en question se fait avec une fiche qui est reliée au conducteur 1, mais qui n'est pas visible sur les figures. On notera également que le terme "transmission" de l'énergie doit s'entendre dans les deux sens, l'organe à desservir par l'électrode pouvant tout aussi bien recevoir de l'énergie que d'en émettre, ce qui est le cas par exemple pour les stimulateurs du type dit "à la demande".

Le conducteur 1 est entouré d'une gaine isolante 2 sur toute sa longueur.

L'électrode comporte également une tête 3 formée par un corps généralement cylindrique avec un axe qui coïncide avec celui du conducteur 1.

L'extrémité arrière de la tête 3 proche de ce dernier comporte un trou longitudinal borgne 4 dans lequel est insérée l'extrémité du conducteur. Il peut y être engagé à force ou y être fixé par soudure au laser ou par sertissage de la tête 3. Un bouchon 5 peut obturer le conducteur 1. On remarquera également qu'à l'arrière, le corps de la tête 3 comporte un renflement 6 délimitant un épaulement annulaire 7 contre lequel est retenue la gaine 2 du conducteur 1. Celle-ci présente par ailleurs à son extrémité une portion 8 de diamètre réduit.

A l'extrémité avant, la tête 3 est également percée d'un trou borgne 9 orienté axialement. Extérieurement, cette extrémité est pourvue d'une portion périphérique 10 dépassant le diamètre de la majeure partie du corps. La surface - enveloppe 11 de cette portion périphérique 10 est une portion de sphère tandis que la face avant 12 de la tête est aplatie et radiale par rapport à l'axe de la tête 3.

Suivant l'invention, la portion périphérique 10 est pourvue d'un réseau de rainures croisées 13a et 13b, les rainures 13a s'étendant parallèlement à l'axe de la tête, tandis que les rainures 13b ont une forme annulaire et s'étendent dans des plans radiaux successifs.

Chaque rainure 13a ou 13b présente un profil transversal en forme de trapèze dont les côtés latéraux s'évasent vers l'extérieur.

De la sorte, la tête de l'électrode permet d'obtenir non seulement une surface de contact très étendue mais également une multitude de cavités dans lesquelles le tissu cardiaque peut croître et adhérer. Une fois cette croissance réalisée, l'électrode est retenue dans tous les sens et plus particulièrement axialement. Pour fixer les idées, lorsque le diamètre hors tout de la surface enveloppe 11 est de 2 mm, la rainure annulaire intermédiaire peut avoir une profondeur de 0,3 mm, l'angle que font les parois de la rainure étant égal à 40°.

Deux trous latéraux 14 sont prévus près du fond de l'orifice borgne 9. Ces trous sont prévus pour laisser passage à de la matière plastique lors de la formation par injection d'un manchon isolant 15 qui s'étend entre la gaine 2 et la portion périphérique 10 de l'électrode. Lors de l'injection, la matière plastique est introduite par l'orifice 9, c'est-à-dire par l'avant de la tête.

Le manchon 15 peut comporter des barbillons 16 connus en soi et s'étendant obliquement vers l'arrière afin d'améliorer encore l'accrochage.

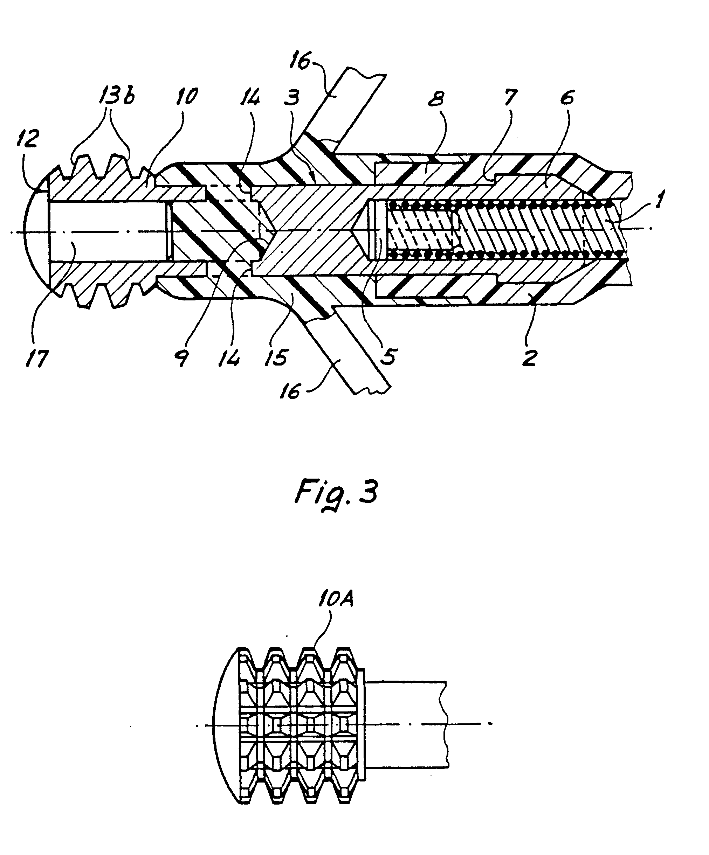

Dans la variante des figures 1 et 2, la matière plastique du manchon 15 remplit la totalité du trou borgne 9. Mais, comme le montre la figure 3, ceci n'est pas forcément le cas. En effet, ici une partie du trou 9 est remplie par un bouchon métallique 17 dont la surface frontale sphérique se raccorde sur la surface - enveloppe 11, comme le fait d'ailleurs la matière plastique dans le cas de la figure 1.

On remarquera que le réseau de rainures croisées 13a et 13b peut être réalisé par de simples opérations d'usinage au tour et à la fraise, ce qui revient à un coût de fabrication réduit. Ces opérations permettent en même temps d'obtenir un fond dans chaque rainure d'une certaine largeur telle que le tissu cardiaque peut venir jusqu'au fond de chaque rainure. La largeur en question peut aller jusqu'à 0,1 mm, par exemple. Toutefois, il est également envisageable de fabriquer la tête par pressage direct de métal selon des méthodes connues.

En ce qui concerne les matériaux, la tête 3 peut être réalisée en platine ou tout autre métal conducteur facilement usinable et compatible avec un bon accrochage et une bonne croissance du tissu. La gaine 2 et le manchon 15 peuvent être réalisés en "Silastic" (marque déposée), par exemple.

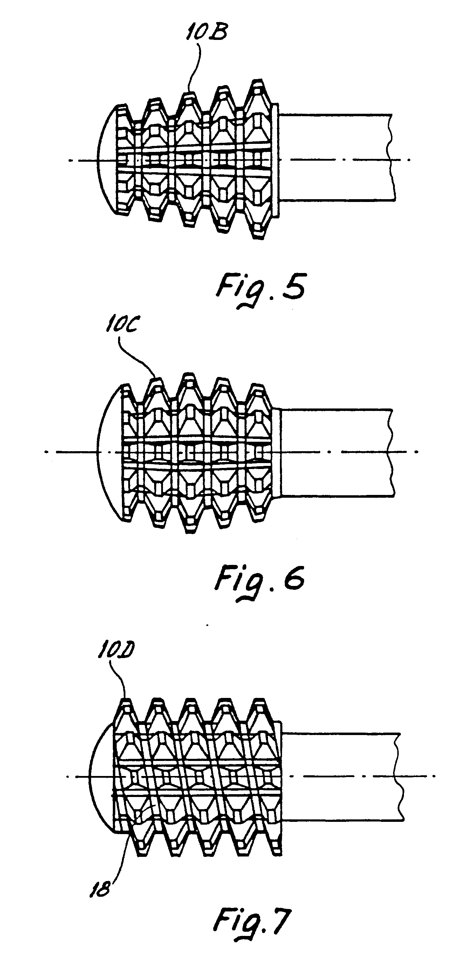

Les rainures 13a et 13b (considérées développées dans un plan) peuvent se croiser à angle droit ou obliquement, ce qui n'a pas beaucoup d'influence sur l'efficacité de l'électrode. Par ailleurs, la portion périphérique 10 peut être modifiée selon les variantes représentées aux figures 4 à 7, qui représentent respectivement une forme cylindrique 10A, une forme troconique 10B se rétrécissant vers l'avant, une forme biconique 10C et enfin une forme de nouveau cylindrique 10D, cependant que le réseau de rainures comporte ici dans le sens circonférentiel une seule rainure 18 en forme d'hélice.

## Revendications

1. Electrode implantable, notamment endocardiaque, destinée à établir une connexion avec un organe du corps, qui comporte un conducteur isolé (1, 2) à l'une des extrémités duquel est prévue une tête conductrice (3) destinée à s'accrocher au tissu à desservir pour y assurer ladite connexion, la surface extérieure (11) de la tête (3) étant de révolution autour de l'axe longitudinal du conducteur isolé, caractérisée en ce que ladite surface extérieure présente un réseau de rainures croisées (13a, 13b, 18) venues de formage dans le corps de la tête.

2. Electrode suivant la revendication 1, caractérisée en ce que chacune des rainures croisées (13a, 13b, 18) présente un profil transversal en trapèze, les parois latérales s'évasant vers l'extérieur.

3. Electrode suivant l'une quelconque des revendications 1 et 2, caractérisée en ce que les rainures considérées développées dans un plan se croisent à angle droit. ·

4. Electrode suivant l'une quelconque des revendications 1 et 2, caractérisée en ce que le réseau de rainures comporte une rainure continue (18) en hélice coaxialement à l'axe de la tête (3).

5. Electrode suivant l'une quelconque des revendications précédentes, caractérisée en ce que ladite surface extérieure (11) revêt la forme d'une portion de sphère ou d'un cylindre à section circulaire, ou est tronconique ou biconique.

6. Electrode suivant l'une quelconque des revendications 1 à 5 comprenant un manchon extérieur (15) isolant se raccordant sur l'isolant dudit conducteur isolé (1, 2), caractérisée en ce que ladite tête (3) comporte un trou borgne (9) à son extrémité avant disposé coaxialement à cette tête et communiquant avec l'extérieur à travers au moins un trou latéral (14) servant lors de la confection dudit manchon (15) par injection à laisser passage à la matière plastique destinée à former le manchon.

## Patentansprüche

1. Implantierbare Elektrode, insbesondere für endokardiale Anwendungen, die zur Herstellung einer Verbindung mit einem Körperorgan bestimmt ist und einen isolierten Leiter (1, 2) umfaßt, an dessen einem Ende ein Leiterkopf (3) angeordnet ist, der sich am Zielgewebe festhaken kann, um die genannte Verbindung zu gewährleisten, wobei die äußere Oberfläche (11) des Kopfes (3) ringförmig um die Längsachse des isolierten Leiters verläuft, dadurch gekennzeichnet, dass die genannte äußere Oberfläche aus einem Netz von Kreuznuten (13a, 13b, 18) besteht, die im Körper des Kopfes eingeformt sind.

2. Elektrode gemäss Anspruch 1, dadurch gekennzeichnet, dass jede dieser Kreuznuten (13a, 13b, 18) ein trapezförmiges Querprofil aufweist, dessen Seitenwände sich nach außen erweitern.

3. Elektrode gemäss einem beliebigen der Ansprüche 1 und 2, dadurch gekennzeichnet, dass sich die genannten Nuten bei Abwicklung in einer Ebene im rechten Winkel kreuzen.

4. Elektrode gemäss einem beliebigen der Ansprüche 1 und 2, dadurch gekennzeichnet, dass das Nutennetz eine durchgehende, wendelförmig koaxial zur Achse des Kopfes (3) verlaufende Nute (18) umfaßt.

5. Elektrode gemäss einem beliebigen der vorstehenden Ansprüche, dadurch gekennzeichnet, dass die genannte äußere Oberfläche (11) die Form eines Kugelabschnitts oder eines Zylinders runden Querschnitts aufweist oder kegelstupfförmig oder doppelkegelig ist.

6. Elektrode gemäss einem beliebigen der Ansprüche 1 bis 5, umfassend eine äußere Isoliermanschette (15), die auf den Isolator des genannten isolierten Leiters (1, 2) aufgesetzt wird, dadurch gekennzeichnet, dass der genannte Kopf (13) ein Sackloch (9) an seinem vorderen Ende aufweist, das koaxial zum Kopf angeordnet und mit der Außenseite über mindestens ein seitliches Loch (14) verbunden ist, welches bei der Fertigung der genannten Manschette (15) während des Spritzvorgangs den Durchfluß des die Manschette bildenden Kunststoffes gestattet.

## Claims

1. Implantable electrode, particularly endocardial electrode intended to establish a connexion with a body part, comprising an insulated conductor (1, 2) at one end of which is provided a conducting head (3) intended to fasten onto an aimed tissue in order to make said connexion, the outer surface (11) of the head (3) being symmetrical about the longitudinal axis of the isolated conductor, characterized in that said outer surface is provided with a network of intersecting grooves (13a, 13b, 18) integrally formed in the bulk of the head.

2. Electrode according to claim 1, characterized in that each of the intersecting grooves (13a, 13b, 18) has a trapezoidal cross-section, of which the lateral walls flare outwardly.

3. Electrode according to any one of claims 1 and 2, characterized in that the grooves when considered developped on a plan interesect at right angles.

4. Electrode according to any one of claims 1 and 2, characterized in that the grooves network comprises a continuous, helical groove (18) wound around the axis of the head (3).

5. Electrode according to any one of the preceding claims, characterized in that said outer surface (11) is of frustospherical or cylindrical or frustoconical or bifrustoconical shape.

6. Electrode according to any one of claims 1 to 5 comprising an outer insulating sleeve (15) which connects with the insulating part of the insulated conductor (1, 2), characterized in that the front end of said head (3) is provided with a recessed hole (9) extending axially through this head and communicating with the outer side through at least one lateral hole (14) which allows the plastic material intended to form the sleeve to pass, when realizing said sleeve (15) by injection.

Fig. 1

Fig. 2

Fig. 3

10B

Fig. 5

10C

Fig. 6

10D

18

Fig. 7